# EUROPÄISCHE PATENTSCHRIFT

(11) **EP 2 344 563 B1**
(45) Veröffentlichungstag und Bekanntmachung des Hinweises auf die Patenterteilung: **14.06.2017**
(21) Anmeldenummer: 09736949.0
(22) Anmeldetag: 19.10.2009
(51) Int. Cl.: C08G 18/73, C08G 18/79, C07C 263/18, C07C 263/20, C07D 229/00, C07D 251/34

(54) **VERFAHREN ZUR HERSTELLUNG FARBLOSER POLYISOCYANATE**
METHOD FOR PRODUCING COLOURLESS POLYISOCYANATES
PROCÉDÉ DE PRÉPARATION DE POLYISOCYANATES INCOLORES

(30) Priorität: 22.10.2008 EP 08167303
(43) Veröffentlichungstag der Anmeldung: 20.07.2011
(73) Patentinhaber: BASF SE, 67056 Ludwigshafen am Rhein (DE)
(72) Erfinder: BINDER, Horst, 68623 Lampertheim (DE); KRONER, Matthias, 67304 Eisenberg (DE); BAYER, Alexander, 67117 Limburgerhof (DE)
(74) Vertreter: BASF IP Association
(86) Internationale Anmeldenummer: PCT/EP2009/063634
(87) Internationale Veröffentlichungsnummer: WO 2010/046327

(56) Entgegenhaltungen:
- EP-A2- 0 569 804
- EP-A2- 0 630 928
- DE-A1-102006 043 464
- US-A- 5 919 887

## Beschreibung

Die vorliegende Erfindung betrifft ein Verfahren zur Herstellung farbreduzierter Polyisocyanate, insbesondere zur Verringerung der Farbe von Polyisocyanaten.

Zur Verringerung der Farbe in Polyisocyanaten nach ihrer Herstellung sind im Stand der Technik verschiedene Verfahren bekannt.

JP 03036975 B offenbart ein Verfahren zur Entfärbung von methylenverbrückten Polyphenylen-Polyisocyanaten, in dem man die Polyisocyanate mit Licht der Wellenlänge 200 bis 700 nm, bevorzugt 300 bis 500 bestrahlt.

Da der Vergilbungsmechanismus bei aromatischen Isocyanaten auf eine Reaktion der aromatischen Amine mit Sauerstoff - es reagieren die benzylischen Wasserstoffatome des Aromaten mit Sauerstoff - zurückzuführen ist, ist die der JP 03036975 B zugrundeliegende Problematik eine andere als die bei der vorliegenden Erfindung: In der vorliegenden Erfindung soll die Farbzahl erstens von Polyisocyanaten und zweitens aliphatischer Isocyanate verringert werden, wohingegen in der JP 03036975 B aromatische Diisocyanate behandelt werden, die nicht durch Oligomerisierung der Isocyanatgruppen weiterbehandelt sind. Der Fachmann kann also nicht von einer Übertragbarkeit der Lehre der JP 03036975 B auf die zu lösende Problematik ausgehen, da der Vergilbungsmechanismus bei aromatischen Isocyanaten naturgemäß ein anderer ist als bei aliphatischen, da es bei letzteren keine benzylische Gruppe existiert.

EP 377177 A1 beschreibt die Nachbehandlung von (cyclo)aliphatischen Polyisocyanaten, die durch Trimerisierung von Diisocyanaten mit Phosphinen als Katalysator hergestellt worden sind, mit Peroxid. Die Mischungen müssen bei diesem Verfahren thermisch nachbehandelt werden.

WO 97/45399 beschreibt die Farbverbesserung von Polyisocyanaten auf Basis Hexamethylendiisocyanat, die durch Phosphine oligomerisiert worden sind, durch Peroxide.

DE 10 2006 043464 beschreibt die Behandlung eines Polyisocyanat mit einem Peroxid oder Hydroperoxid.

EP 630928 A2 beschreibt die verbesserte Farbzahl und Lagerstabilität von Polyisocyanaten, die durch Ammoniumsalze oder Metallsalze als Katalysator hergestellt worden sind, durch Zusatz von organischen Persäuren. Die Säurefunktion dient gleichzeitig der Zerstörung des Katalysators um die Reaktion abzubrechen.

Die Behandlung von Polyisocyanat mit Ozon, sauerstoff- oder ozonhaltigen Gasgemischen ist bekannt aus EP 569804.

Nachteilig an diesen Verfahren ist, daß das zugesetzte Peroxid oder Hydroperoxid bzw. die Abbauprodukte der farbgebenden Komponenten im Produkt verbleiben.

Aufgabe der vorliegenden Erfindung war es, ein Verfahren zu entwickeln, mit dem eine bereits gebildete Farbe in Polyisocyanaten ohne Zusatz von systemfremden Stoffen entfernt werden kann.

Die Aufgabe wurde gelöst durch ein Verfahren zur Herstellung farbreduzierter (cyclo)aliphatischer Polyisocyanate, in dem man das Polyisocyanat auf Basis (cyclo)aliphatischer Diisocyanate mit höherer als gewünschter Farbzahl mit Licht der Wellenlänge von 200 bis 600 nm mit einer Strahlungsdose von 20 bis 40000 J/cm² in diesem Wellenlängenbereich bestrahlt, die ausreicht um die Farbzahl um mindestens 1 Hazen zu verringern, wobei das man Verfahren ohne Zusatz von Ozon, Sauerstoff oder ozonhaltigen Gasgemischen, organischen Persäuren, Peroxid oder Hydroperoxid, durchführt und wobei die Farbzahl nach DIN EN 1557 bestimmt wird.

Zudem wurde gefunden, daß die erfindungsgemäß behandelten Polyisocyanate eine geringere Viskosität aufweisen als nicht behandelte. Dies hat zur Folge, daß bei den erfindungsgemäß behandelten Polyisocyanaten eine geringere Menge Lösungsmittel zur Einstellung der Verarbeitungsviskosität ausreichend ist.

Als Polyisocyanate sind für das erfindungsgemäße Verfahren besonders Polyisocyanate auf Basis (cyclo)aliphatischer Diisocyanate geeignet.

Der Begriff (cyclo)aliphatische steht in dieser Schrift kurz für cycloaliphatisch oder aliphatisch.

Cycloaliphatische Isocyanate sind solche, die mindestens ein cycloaliphatisches Ringsystem enthalten.

Aliphatische Isocyanate sind solche, die ausschließlich gerade oder verzweigte Ketten enthalten, also acyclische Verbindungen.

Die erfindungsgemäß einsetzbaren Polyisocyanate weisen keine aromatischen Gruppen auf.

Bei den monomeren Isocyanaten handelt es sich bevorzugt um Diisocyanate, die genau zwei Isocyanatgruppen tragen. Es könnte sich aber prinzipiell auch um Monoisocyanate mit einer Isocyanatgruppe handeln, diese sind aber weniger bevorzugt.

Es kommen prinzipiell auch höhere Isocyanate mit im Mitte! mehr als 2 Isocyanatgruppen in Betracht, diese sind aber weniger bevorzugt. Dafür eignen sich beispielsweise Triisocyanate wie Triisocyanatononan oder 2'-Isocyanatoethyl-(2,6-diisocyanatohexanoat), oder die Gemische aus Di-, Tri- und höheren Polyisocyanaten.

Die monomeren Isocyanate weisen im wesentlichen keine Umsetzungsprodukte der Isocyanatgruppen mit sich selbst auf.

Bei den monomeren Isocyanaten handelt es sich bevorzugt um Isocyanate mit 4 bis 20 C-Atomen. Beispiele für übliche aliphatische Diisocyanate sind Tetramethylendiisocyanat, 1,5-Pentamethylendiisocyanat, Hexamethylendiisocyanat (1,6-Diisocyanatohexan), Octamethylendiisocyanat, Decamethylendiisocyanat, Dodecamethylendiisocyanat, Tetradecamethylendiisocyanat, Derivate des Lysindiisocyanates, (z.B. Methyl- oder Ethyl-2,6-diisocyanatohexanoat), Trimethylhexandiisocyanat oder Tetramethylhexandiisocyanat. Beispiele für cycloaliphatische Diisocyanate sind 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-methylcyclohexan sowie 3 (bzw. 4), 8 (bzw. 9)-Bis(isocyanatomethyl)-tricyclo[5.2.1.0^{2.6}]decan-Isomerengemische.

Besonders bevorzugte Diisocyanate sind 1,6-Hexamethylendiisocyanat, 1,3-Bis(isocyanatomethyl)cyclo-hexan und Isophorondiisocyanat, ganz besonders bevorzugt sind Isophorondiisocyanat und 1,6-Hexamethylendiisocyanat, insbesondere bevorzugt ist Isophorondiisocyanat.

Es können auch Gemische der genannten Isocyanate vorliegen.

Isophorondiisocyanat liegt zumeist als ein Gemisch, und zwar der cis- und trans-Isomere vor, in der Regel im Verhältnis von ca. 60:40 bis 80:20 (w/w), bevorzugt im Verhältnis von ca. 70:30 bis 75:25 und besonders bevorzugt im Verhältnis von ca. 75:25.

Der Gehalt an isomeren Verbindungen im Diisocyanat spielt für das erfindungsgemäße Verfahren keine entscheidende Rolle. So kann 1,6-Hexamethylendiisocyanat beispielsweise einen geringen Anteil an 2- und/oder 3-Methyl-1,5-pentamethylendiisocyanat enthalten.

Für die vorliegende Erfindung können Polyisocyanate sowohl auf Basis solcher Diisocyanate eingesetzt werden, die durch Phosgenierung der korrespondierenden Amine erhalten wird, als auch solche, die ohne die Verwendung von Phosgen, d. h. nach phosgenfreien Verfahren, hergestellt werden. Nach Angaben der EP-A-0 126 299 (US 4 596 678), EP-A-126 300 (US 4 596 679) und EP-A-355 443 (US 5 087 739) beispielsweise können (cyclo)aliphatische Diisocyanate, wie 1,6-Hexamethylendiisocyanat (HDI), hergestellt werden durch Umsetzung der (cyclo)aliphatischen Diamine mit beispielsweise Harnstoff und Alkoholen zu (cyclo)aliphatischen Biscarbaminsäureestern und deren thermische Spaltung in die entsprechenden Diisocyanate und Alkohole. Die Synthese erfolgt meist kontinuierlich in einem Kreislaufverfahren und gegebenenfalls in Gegenwart von N-unsubstituierten Carbaminsäureestern, Dialkylcarbonaten und anderen aus dem Reaktionsprozeß zurückgeführten Nebenprodukten. So erhaltene Diisocyanate weisen in der Regel einen sehr geringen oder sogar nicht meßbaren Anteil an chlorierten Verbindungen auf, was zu günstigen Farbzahlen der Produkte führen kann. Es stellt einen weiteren Vorteil der vorliegenden Erfindung dar, daß das erfindungsgemäße Verfahren auf aliphatische Diisocyanate unabhängig von deren Herstellung ist, d.h. unabhängig davon, ob die Herstellung über eine Phosgenierung oder ein phosgenfreies Verfahren erfolgt ist.

In einer Ausführungsform der vorliegenden Erfindung weist das Diisocyanat einen Gesamtgehalt an hydrolysierbarem Chlor von weniger als 200 ppm auf, bevorzugt von weniger als 120 ppm, besonders bevorzugt weniger als 80 ppm, ganz besonders bevorzugt weniger als 50 ppm, insbesondere weniger als 15 ppm und speziell weniger als 10 ppm. Dies kann beispielsweise gemessen werden durch die ASTM-Vorschrift D4663-98. Es können aber selbstverständlich auch Diisocyanate mit einem höheren Chlorgehalt eingesetzt werden, beispielsweise bis zu 500 ppm.

Selbstverständlich können auch Gemische aus Diisocyanat, das durch Umsetzung des korrespondierenden Diamins mit beispielsweise Harnstoff und Alkoholen und Spaltung der erhaltenen Biscarbaminsäureester erhalten worden ist, mit solchem Diisocyanat, das durch Phosgenierung des korrespondierenden Amins erhalten worden sind, eingesetzt werden.

Bei den Polyisocyanaten auf Basis dieser Diisocyanate handelt es sich bevorzugt um folgende Verbindungen:
1) Isocyanuratgruppen aufweisende Polyisocyanate von aliphatischen und/oder cycloaliphatischen Diisocyanaten. Besonders bevorzugt sind hierbei die entsprechenden aliphatischen und/oder cycloaliphatischen Isocyanato-Isocyanurate und insbesondere die auf Basis von Hexamethylendiisocyanat und/oder Isophorondiisocyanat. Bei den dabei vorliegenden Isocyanuraten handelt es sich insbesondere um Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im allgemeinen einen NCO-Gehalt von 10 bis 30 Gew.-%, insbesondere 15 bis 25 Gew.-% und eine mittlere NCO-Funktionalität von 2,6 bis 8.
2) Uretdiongruppen aufweisende Polyisocyanate mit aliphatisch und/oder cycloaliphatisch gebundenen Isocyanatgruppen, vorzugsweise aliphatisch und/oder cycloaliphatisch gebundenen und insbesondere die von Hexamethylendiisocyanat oder Isophorondiisocyanat abgeleiteten. Bei Uretdiondiisocyanaten handelt es sich um cyclische Dimerisierungsprodukte von Diisocyanaten.
   Die Uretdiongruppen aufweisenden Polyisocyanate werden im Rahmen dieser Erfindung im Gemisch mit anderen Polyisocyanaten, insbesondere den unter 1) genannten, erhalten. Dazu können die Diisocyanate unter Reaktionsbedingungen umgesetzt werden, unter denen sowohl Uretdiongruppen als auch die anderen Polyisocyanate gebildet werden, oder zunächst die Uretdiongruppen gebildet und diese anschließend zu den anderen Polyisocyanaten umgesetzt werden oder die Diisocyanate zunächst zu den anderen Polyisocyanaten und diese anschließend zu Uretdiongruppen-haltigen Produkten umgesetzt werden.
3) Urethan- und/oder Allophanatgruppen aufweisende Polyisocyanate mit aliphatisch oder cycloaliphatisch gebundenen Isocyanatgruppen, wie sie beispielsweise durch Umsetzung von überschüssigen Mengen an Diisocyanat, beispielsweise Hexamethylendiisocyanat oder Isophorondiisocyanat, mit ein- oder mehrwertigen Alkoholen. Diese Urethan- und/oder Allophanatgruppen aufweisenden Polyisocyanate haben im allgemeinen einen NCO-Gehalt von 12 bis 24 Gew.-% und eine mittlere NCO-Funktionalität von 2,1 bis 4,5. Solche Urethan- und/oder Allophanatgruppen aufweisenden Polyisocyanate können unkatalysiert oder bevorzugt in Gegenwart von Katalysatoren, wie beispielsweise Ammoniumcarboxylaten oder -hydroxiden, oder Allophanatisierungskatalysatoren, z.B. Zn-(II)-Verbindungen, jeweils in Anwesenheit von ein-, zwei- oder mehrwertigen, bevorzugt einwertigen Alkoholen, hergestellt werden. Die Urethan- und/oder Allophanatgruppen aufweisenden Polyisocyanate können auch im Gemisch mit anderen Polyisocyanaten, insbesondere den unter 1) genannten, hergestellt werden.
4) Uretonimin-modifizierte Polyisocyanate.
5) Carbodiimid-modifizierte Polyisocyanate.
6) Hyperverzweigte Polyisocyanate, wie sie beispielsweise bekannt sind aus der DE-A1 10013186 oder DE-A1 10013187.
7) Polyurethan-Polyisocyanat-Präpolymere, aus Di- und/oder Polyisocyanaten mit Alkoholen.
8) Polyharnstoff-Polyisocyanat-Präpolymere.
9) Hydrophil modifizierte Polyisocyanate, d.h. Polyisocyanate, die neben den unter 1-10 beschriebenen Gruppen solche enthalten, die formal durch Addition von Molekülen mit NCO-reaktiven Gruppen und hydrophilierenden Gruppen an die Isocyanatgruppen obiger Moleküle entstehen. Bei letzteren handelt es sich um nichtionische Gruppen wie Alkyl-Polyethylenoxid und/oder ionische, welche beispielsweise von Phosphorsäure, Phosphonsäure, Schwefelsäure oder Sulfonsäure, bzw. ihren Salzen abgeleitet sind.
10) Iminooxadiazindiongruppen enthaltende Polyisocyanate, vorzugsweise von Hexamethylendiisocyanat oder Isophorondiisocyanat abgeleitet. Solche Iminooxadiazindiongruppen enthaltenden Polyisocyanate sind aus Diisocyanaten mittels spezieller Katalysatoren herstellbar.

In einer bevorzugten Ausführungsform der vorliegenden Erfindung enthält das Polyisocyanat mindestens eine Gruppierung, ausgewählt aus der Gruppe bestehend aus Isocyanuraten, Urethanen und Allophanaten, bevorzugt aus der Gruppe bestehend aus Isocyanuraten und Allophanaten, besonders bevorzugt handelt es sich um ein isocyanuratgruppenhaltiges Polyisocyanat.

In einer besonders bevorzugten Ausführungsform handelt es sich bei dem Polyisocyanat um Isocyanuratgruppen enthaltende Polyisocyanate von 1,6-Hexamethylendiisocyanat und/oder Isophorondiisocyanat, ganz besonders bevorzugt um ein Isocyanuratgruppen enthaltendes Polyisocyanat auf Basis von Isophorondiisocyanat.

In dieser Schrift wird die Viskosität bei 23 °C gemäß DIN EN ISO 3219/A.3 in einem Kegel-Platte-System mit einem Geschwindigkeitsgefälle von 250 s⁻¹ angegeben, falls nicht anders vermerkt.

Die oben aufgeführten Polyisocyanate können auch zumindest teilweise in blockierter Form vorliegen.

Zur Blockierung eingesetzte Verbindungsklassen sind beschrieben in D. A. Wicks, Z. W. Wicks, Progress in Organic Coatings, 36, 148-172 (1999), 41, 1-83 (2001) sowie 43, 131-140 (2001).

Beispiele für zur Blockierung eingesetzte Verbindungsklassen sind Phenole, Imidazole, Triazole, Pyrazole, Oxime, N-Hydroxyimide, Hydroxybenzoesäureester, sekundäre Amine, Lactame, CH-acide cyclische Ketone, Malonsäureester oder Alkylacetoacetate.

Bevorzugt kann das erfindungsgemäße Verfahren zur Farbverbesserung solcher Polyisocyanate eingesetzt werden, die durch Umsetzung von monomeren Isocyanaten durch Einwirkung von mindestens einem Katalysator erhalten worden sind, der mindestens ein quartäres Stickstoffatom aufweist, besonders bevorzugt von Katalysatoren, die quartäre Ammoniumionen aufweisen.

Ganz besonders bevorzugt handelt es sich bei den Ammoniumionen um solche der Formel

R¹R²R³R⁴N⁺ Y⁻

worin
R¹, R², R³ und R⁴ jeweils unabhängig voneinander gegebenenfalls durch Aryl, Alkyl, Aryloxy, Alkyloxy, Heteroatome und/oder Heterocyclen substituiertes C₁ - C₂₀-Alkyl, C₆ - C₁₂-Aryl oder C₅ - C₁₂-Cycloalkyl und Y⁻ ein beliebiges Anion bedeuten.

Bevorzugt sind darin R¹, R², R³ und R⁴ jeweils unabhängig voneinander ausgewählt aus der Gruppe bestehend aus Methyl, Ethyl, n-Butyl, Octyl, Benzyl, 2-Hydroxyethyl und 2-Hydroxypropyl.

In einer bevorzugten Ausführungsform sind die Anionen Y⁻ darin ausgewählt aus der Gruppe bestehend aus Fluorid, Hydroxid, Carboxylat, Carbonat und α-Hydroxycarboxylat, besonders bevorzugt ausgewählt aus der Gruppe bestehend aus Hydroxid, Carboxylat, Carbonat und α-Hydroxycarboxylat und ganz besonders bevorzugt ausgewählt aus der Gruppe bestehend aus Hydroxid und Carboxylat.

Die Carboxylationen sind dabei aromatisch, aliphatisch oder cycloaliphatisch, bevorzugt aromatisch oder aliphatisch und besonders bevorzugt aliphatisch und weisen bis zu 12 Kohlenstoffatome, bevorzugt 1 bis 8 Kohlenstoffatome auf.

Beispiele für besonders bevorzugte Carboxylationen sind Formiat, Acetat, Propionat und 2-Ethylhexanoat, ganz besonders bevorzugt Formiat und 2-Ethylhexanoat, insbesondere 2-Ethylhexanoat.

In weiteren Ausführungsformen handelt es sich bei den Katalysatoren um folgende Typen:
- quartäre Ammoniumcarboxylate des in den US-Patenten 4,454,317 und 4,801,663 beschriebenen Typs;
- quartäre Ammoniumphenolate mit einer zwitterionischen Struktur des im US-Patent 4,335,219 beschriebenen Typs;
- Ammoniumphosphonate und -phosphate des im US-Patent 4,499,253 beschriebenen Typs;
- Gemische aus Alkalimetallfluoriden und quartären Ammonium- oder Phosphoniumsalzen, wie sie in EP-A 355479; EP 798299 B1, oder EP 896009 B1 zur Herstellung von Mischungen von Isocyanurat mit asymmetrischen Isocyanuraten, Iminooxodiazindion, beschrieben sind,
- Ammonium α-Hydroxycarboxylate, wie beschrieben in WO 2005/087828,
- Ammonium carbonate oder -betaine, wie beschrieben in EP 668271,
- quartäre Hydroxyalkylammoniumverbindungen der Formel

   R⁵,R⁶,R⁷N^{⊕}-CH₂-CH(OH)-R^{8 ⊖}O-(CO)-R⁹

   als Katalysator gemäß DE-A-26 31 733 (US-A-4 040 992).

Darin handelt es sich bei den Resten R⁵ bis R⁹ unabhängig voneinander um Wasserstoff, C₁ bis C₄-Alkyl und Benzyl und bei R⁹ zusätzlich dazu um C₅- bis C₈-Alkyl.

Besonders geeignet als Katalysatoren für das Verfahren sind quartäre Ammoniumsalze entsprechend der Formel 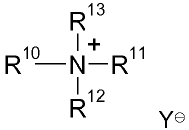 mit
Y^{⊖}= Carboxylat (R¹⁴COO⁻), Fluorid (F-), Carbonat (R¹⁴O(CO)O⁻) oder Hydroxid (OH-),
wie sie für Y⁻ = OH⁻ im US-Patent 4,324,879 und in den Deutschen Offenlegungsschriften 2,806,731 und 2,901,479 beschrieben sind.

Bevorzugt handelt es sich bei dem Rest Y^{⊖} um ein Carboxylat, Carbonat oder Hydroxid, besonders bevorzugt um ein Carboxylat oder Hydroxid und ganz besonders bevorzugt um ein Carboxylat.

R¹⁴ ist darin Wasserstoff, C₁ bis C₂₀-Alkyl, C₆ bis C₁₂-Aryl oder C₇ bis C₂₀-Arylalkyl, das jeweils optional substituiert sein kann.

Bevorzugt ist R¹⁴ Wasserstoff oder C₁ bis C₈-Alkyl.

Wird ein Katalysator mit einem Hydroxidion als Anion eingesetzt, so ist es bevorzugt, die Reaktion bei einem verminderten Gehalt an Kohlenstoffdioxid (CO₂) durchzuführen, beispielsweise von weniger als 20 ppm, bevorzugt von weniger als 10 und besonders bevorzugt von weniger als 5 ppm, wie beschrieben in der EP 330966 A2.

Bevorzugte quartäre Ammoniumsalze sind diejenigen, bei denen die Reste R¹⁰ bis R¹³ gleiche oder unterschiedliche Alkylgruppen mit 1 bis 20, vorzugsweise 1 bis 4, Kohlenstoffatomen darstellen, die gegebenenfalls durch Hydroxyl- oder Phenylgruppen substituiert sind.

Zwei der Reste R¹⁰ bis R¹³ können auch zusammen mit dem Stickstoffatom und gegebenenfalls einem weiteren Stickstoff- oder Sauerstoffatom einen heterocyclischen, fünf-, sechs- oder siebengliedrigen Ring bilden. Die Reste R¹⁰ bis R¹² können in jedem Falle auch Ethylenreste darstellen, die zusammen mit dem quartären Stickstoffatom und einem weiteren tertiären Stickstoffatom eine bicyclische Triethylendiaminstruktur bilden, vorausgesetzt, daß der Rest R¹³ dann eine Hydroxyalkylgruppe mit 2 bis 4 Kohlenstoffatomen darstellt, bei der die Hydroxylgruppe vorzugsweise in der 2-Stellung zu dem quartären Stickstoffatom angeordnet ist. Der hydroxysubstituierte Rest oder die hydroxysubstituierten Reste können auch andere Substituenten enthalten, beispielsweise C₁- bis C₄-Alkyloxy-Substituenten.

Dabei können die Ammoniumionen auch Teil eines ein- oder mehrgliedrigen Ringsystems sein, beispielsweise abgeleitet von Piperazin, Morpholin, Piperidin, Pyrrolidin, Chinuclidin oder Di-aza-bicyclo-[2.2.2]-octan.

Beispiele für 1 bis 20 Kohlenstoffatome aufweisende Gruppen R¹⁰ bis R¹³ sind unabhängig voneinander Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, 2-Hydroxyethyl, 2-Hydroxypropyl, 3-Hydroxypropyl, 4-Hydroxybutyl, 6-Hydroxyhexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Methylnaphthyl, Isopropylnaphthyl, Chlornaphthyl, 2,6-Dimethylphenyl, 2,4,6-Trimethylphenyl, 2,6-Dimethoxyphenyl, 2,6-Dichlorphenyl, Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Chlorcyclohexyl, Dichlorcyclohexyl, Dichlorcyclopentyl, Norbornyl oder Norbornenyl.

Bevorzugt sind unabhängig voneinander die Reste R¹⁰ bis R¹³ C₁ bis C₄-Alkyl. R¹³ kann zusätzlich Benzyl sein oder ein Rest der Fomel 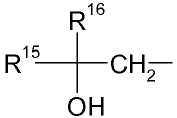 worin R¹⁵ und R¹⁶ unabhängig voneinander Wasserstoff oder C₁ bis C₄-Alkyl sein kann.

Besonders bevorzugte Reste R¹⁰ bis R¹³ sind unabhängig voneinander Methyl, Ethyl und n-Butyl und für R¹³ zusätzlich Benzyl, 2-Hydroxyethyl und 2-Hydroxypropyl.

Bevorzugt können für das erfindungsgemäße Verfahren folgende Katalysatoren eingesetzt werden:
Quarternäre Ammoniumhydroxide, vorzugsweise N,N,N-Trimethyl-N-benzylammoniumhydroxid und N,N,N-Trimethyl-N-(2-hydroxypropyl)-ammoniumhydroxid, gemäß DE-A-38 06 276, unter der Voraussetzung, daß das Verfahren dann bevorzugt unter einem verringerten Gehalt an Kohlenstoffdioxid durchgeführt wird.

Hydroxyalkyl substituierte quarternäre Ammoniumhydroxide gemäß EP-A-10 589 (US-A-4 324 879).

In dieser Schrift bedeuten
gegebenenfalls durch Aryl, Alkyl, Aryloxy, Alkyloxy, Heteroatome und/oder Heterocyclen substituiertes C₁ - C₂₀-Alkyl beispielsweise Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Decyl, Dodecyl, Tetradecyl, Hetadecyl, Octadecyl, Eicosyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl,
gegebenenfalls durch Aryl, Alkyl, Aryloxy, Alkyloxy, Heteroatome und/oder Heterocyclen substituiertes C₆ - C₁₂-Aryl beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, E-thoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch Aryl, Alkyl, Aryloxy, Alkyloxy, Heteroatome und/oder Heterocyclen substituiertes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,
zweiwertige C₂ bis C₉-Alkylenreste, die auch Bestandteil eines Arylen- oder Cycloalkylenrestes sein können, sind beispielsweise 1,2-Ethylen, 1,2-Propylen, 1,3-Propylen, 1,6-Hexylen, 2,2,4-Trimethylhexylen, 1,4-Cyclohexylen, Isopropyliden-1,4-dicyclohexylen, 1,2- 1,3- oder 1,4-Phenylen, 4,4'-Biphenylen, 4,4'-Bisphenylmethylen, 1,3-, 1,4- oder 1,5-Naphthylen, 3,3'-Dimethyl-4,4'-diphenylen, 3,3'-Dichlor-4,4'-diphenylen, 2,4- oder 2,6-Pyridyl, 1,4-Anthrachinondiyl, m- oder p-Toluylen, 4,6-Dimethyl-1,3-phenylen, 4,6-Dichloro-1,3-phenylen, 5-Chloro-1,3-phenylen, 5-Hydroxy-1,3-phenylen, 5-Methoxy-1,3-phenylen, 2,3-Dimethyl-1,4-phenylen m- oder p-Xylylen, Methylen-di-p-phenylen, Isopropyliden-di-p-phenylen, Thio-di-p-phenylen, Dithio-di-p-phenylen, Sulfo-di-p-phenylen, Carbonyl-di-p-phenylen, und
C₁ bis C₄-Alkyl beispielsweise Methyl, Ethyl, Propyl, Isopropyl, n-Butyl, sec-Butyl oder tert.-Butyl, bevorzugt Methyl, Ethyl oder n-Butyl, besonders bevorzugt Methyl oder Ethyl und ganz besonders bevorzugt Methyl.

Die Herstellung dieser quartären Ammoniumkatalysatoren erfolgt in bekannter Weise, beispielsweise durch Umsetzen eines tertiären Amins mit einem Alkylenoxid in einem wäßrig-alkoholischen Medium (vgl. US-Patent 3,995,997, Spalte 2, Zeilen 19-44).

Beispiele für geeignete tertiäre Amine sind Trimethylamin, Tributylamin, 2-Dimethylaminoethanol, Triethanolamin, Dodecyldimethylamin, N,N-Dimethylcyclohexylamin, N-Methylpyrrolidin, N-Methylmorpholin und 1,4-Diazabicyclo[ 2.2.2]octan. Beispiele für geeignete Alkylenoxide sind Ethylenoxid, Propylenoxid, 1,2-Butylenoxid, Styroloxid und Methoxy-, Ethoxy- oder Phenoxypropylenoxid.

Die meistbevorzugten Katalysatoren sind N-(2-Hydroxypropyl)-N,N,N-trimethylammonium-2-ethylhexanoat (DABCO TMR®) und N-(2-Hydroxypropyl)-N,N,N-trimethylammonium-2-formiat (DABCO TMR®-2) der Firma Air Products.

Weiterhin bevorzugt sind solche Trimerisierungskatalysatoren wie sie bekannt sind aus der DE 10 2004 012571 A1, dort besonders Absatz [0017] bis [0027], sowie aus der EP-A1 668 271, dort besonders von S. 4, Z. 16 bis S. 6, Z. 47, was hiermit Bestandteil der vorliegenden Offenbarung sei.

Die Katalysatoren werden im allgemeinen in Mengen bis zu 1000 Gew.-ppm, vorzugsweise etwa 5 bis 500 Gew.ppm, besonders bevorzugt 10 bis 100 Gew.ppm, bezogen auf das eingesetzte Isocyanat, verwendet.

Die Katalysatoren können in reiner Form oder in Lösung verwendet werden. Zur besseren Handhabung kann der Katalysator in einem geeigneten Lösungsmittel gelöst werden. Es eignen sich hierzu beispielsweise Alkohole, Diole, Ketone, Ether und Ester. Die in dieser Schrift aufgeführten Lösungsmittel, die gegenüber Isocyanatgruppen inert sind, sind als Lösungsmittel geeignet, je nach dem Typ des Katalysators. Dimethylformamid oder Dimethylsulfoxid können ebenfalls als Lösungsmittel für die Katalysatoren verwendet werden.

Die Herstellung der Polyisocyanate ist erfindungsgemäß nicht beschränkt. Beispielsweise kann sie folgendermaßen erfolgen:
a) Zunächst erfolgt die Umsetzung des monomeren Isocyanats in Gegenwart eines Katalysators.

Der Schritt a) kann batchweise oder kontinuierlich durchgeführt werden und kann in einer typischen Ausführungsform beispielsweise folgendermaßen durchgeführt werden:
Im Schritt a) findet die Reaktion der Reaktanden in mindestens einer Reaktionszone statt.

Die mittlere Gesamtverweilzeit in Stufe a) kann bis zu 7 Stunden betragen, bevorzugt bis zu 90 Minuten, besonders bevorzugt bis zu 60 Minuten, ganz besonders bevorzugt bis zu 30 Minuten und insbesondere bis zu 20 Minuten.

Die mittlere Gesamtverweilzeit in Stufe a) beträgt in der Regel mindestens 2 Minuten, bevorzugt mindestens 5 Minuten, besonders bevorzugt mindestens 10 Minuten, ganz besonders bevorzugt mindestens 15 Minuten in und insbesondere mindestens 20 Minuten.

Die Reaktionszone kann rückvermischt oder nicht rückvermischt sein, auch Kombinationen davon sind denkbar.

Bei den Reaktionszonen kann es sich beispielsweise um eine Hintereinanderschaltung von mehreren Rührkesseln handeln (Rührkesselkaskade) oder um mindestens einen Rührkessel, der durch eine geeignete Aufteilung des Reaktionsvolumens beispielsweise durch Trennbleche in mehrere Zonen unterteilt ist (kaskadierter Rührkessel) oder Kombinationen davon.

Die Temperatur im rückvermischten Reaktorsystem beträgt im allgemeinen zwischen 40 °C und 170 °C, bevorzugt zwischen 45 °C und 160 °C, besonders bevorzugt zwischen 50 und 150 °C und ganz besonders bevorzugt zwischen 55 und 140 °C.

Die Katalysatoren werden im allgemeinen in Mengen bis zu 1000 Gew.-ppm, vorzugsweise etwa 5 bis 500 Gew.ppm, besonders bevorzugt 10 bis 100 Gew.ppm, bezogen auf das eingesetzte Isocyanat, verwendet.
b) bei Erreichen des gewünschten Umsatzes im Schritt a) wird mindestens eine Verbindung in das Reaktionsgemisch zugegeben, die den Katalysator in Schritt a) zu desaktivieren vermag.

Der Umsatz kann in Abhängigkeit vom eingesetzten Isocyanat unterschiedlich gewählt werden. Im allgemeinen wird ein Umsatz von 5 bis 60% (bezogen auf den NCO-Gehalt vor der Reaktion) angestrebt, bevorzugt auf 5 bis 35%.

Zur Stoppung in Schritt b) wird das Desaktivierungsmittel in einem molaren Verhältnis von beispielsweise 0,5 bis 20, besonders bevorzugt von 0,6 bis 3, ganz besonders bevorzugt 0,8 bis 2, bezogen auf die eingesetzte Katalysatormenge eingesetzt.

Als Desaktivierungsmittel eignen sich prinzipiell anorganische Säuren, wie z.B. Chlorwasserstoff, phosphorige Säure oder Phosphorsäure, Carbonsäurehalogenide, wie z.B. Acetylchlorid oder Benzoylchlorid, Sulfonsäuren oder-ester, wie z.B. Methansulfonsäure, p-Toluolsulfonsäure, p-Toluolsulfonsäuremethyl- oder -ethylester, m-Chlorperbenzoesäure und vorzugsweise Dialkylphosphate wie z.B. Di-2-ethylhexylphosphat und Dibutylphosphat.

Denkbar ist auch der Einsatz von Carbamatverbindungen, wie beschrieben in der WO 2008/068198.

Die Zugabe des Stoppungsmittels erfolgt in der Regel bei der Reaktionstemperatur, kann aber auch bei höherer oder niedrigerer Temperatur erfolgen, beispielsweise bis zu 30 °C niedriger, bevorzugt bis zu 20 °C niedriger und besonders bevorzugt bis zu 10 °C niedriger.

Alternativ kann das Reaktionsgemisch aus Schritt a) auch dadurch gestoppt oder die Stoppung vervollständigt werden, indem der Katalysator thermisch desaktiviert wird. Dafür sind solche Katalysatoren geeignet, die an Ammoniumgruppen gebundene β-Hydroxyalkylgruppen aufweisen. Dazu wird die Temperatur kurzfristig auf mindestens 100 °C, bevorzugt mindestens 110 °C und besonders bevorzugt mindestens 120 °C erhöht, bevorzugt durch Durchleiten durch einen Destillationsapparat, wie in Schritt c) beschrieben.
c) Abtrennen des unumgesetzten Isocyanats (D) aus dem so erhaltenen Reaktionsgemisch,

Das so hergestellte Polyisocyanate enthaltende Reaktionsgemisch wird abschließend in einem Schritt c) in an sich bekannter Weise, beispielsweise durch Dünnschichtdestillation, bei einer Temperatur von 90 bis 220 °C, gegebenenfalls im Vakuum, gegebenenfalls zusätzlich mit Durchleiten von inertem Strippgas, von gegebenenfalls vorhandenen Lösungs- oder Verdünnungsmittel und/oder vorzugsweise von überschüssigen, nicht-umgesetzten Isocyanaten befreit werden, so daß die Isocyanuratgruppen aufweisenden Polyisocyanate mit einem Gehalt an monomeren Isocyanaten von z.B. unter 1,0 Gew.-%, vorzugsweise unter 0,5 Gew.-%, besonders bevorzugt unter 0,3, ganz besonders bevorzugt unter 0,2 und insbesondere nicht mehr als 0,1 Gew% erhältlich sind.

Als Apparate dafür dienen Flash-, Fallfilm-, Dünnschicht- und/oder Kurzwegverdampfer, denen gegebenenfalls eine kurze Kolonne aufgesetzt sein kann.

Die Destillation erfolgt in der Regel bei einem Druck zwischen 0,1 und 300 hPa, bevorzugt unter 200 hPa und besonders bevorzugt unter 100 hPa.

In einer bevorzugten Ausführungsform wird die Destillation mehrstufig durchgeführt in beispielsweise 2 bis 5 Stufen, bevorzugt 2 bis 4 Stufen und besonders bevorzugt 3 bis 4 Stufen.

Dabei wird der Druck vorteilhafterweise von Stufe zu Stufe abgesenkt, beispielsweise beginnend bei 300 - 500 hPa über 100 bis 300 hPa auf 10 bis 100 hPa und anschließend auf 0,1 bis 10 hPa.

Die Temperatur in den einzelnen Destillationsstufen beträgt jeweils von 90 bis 220 °C.

Vorteilhafterweise wird die erste Stufe in einem einfachen Apparat durchgeführt, beispielsweise einem Umlauf-, Flash- oder Kerzenverdampfer und die nachfolgenden Stufen in komplizierteren Apparaten, beispielsweise in Fallfilmverdampfern, Dünnschichtverdampfern, beispielsweise Sambay®- oder Luwaverdampfer, oder Kurzwegverdampfern. Dabei ist es vorteilhaft, apparative Maßnahmen zu ergreifen, durch die die Verweilzeit der Ströme und damit deren thermische Belastung verringert wird, beispielsweise Verzicht auf Zwischenbehälter oder Vorlagebehälter, kurze Rohrwege oder kleinstmögliche Ausführung der Sumpfvolumina.

Das abgetrennte Destillat von monomerem Isocyanat wird bevorzugt in die Stufe a) zurückgeführt und von neuem, ergänzt um frisch zugeführtes Isocyanat, in die Reaktion eingesetzt.

Im erfindungsgemäßen Verfahren bestrahlt man das Polyisocyanat mit Licht der Wellenlänge von 200 bis 600 nm, bevorzugt 210 bis 500 nm, besonders bevorzugt 220 bis 450 und ganz besonders bevorzugt 220 bis 420 nm.

Bevorzugt sollte die eingesetzte Strahlungsquelle zumindest ein Emissionsmaximum in diesem Wellenlängenbereich aufweisen.

Die Energiedosis ist nach oben prinzipiell nicht begrenzt. Sie ist ausreichend, wenn die gewünschten Farbzahlen erreicht sind.

Die Energiedosis ist ausreichend, um die Farbzahl um mindestens 1 Hazen zu verringern, bevorzugt um mindestens 2 Hazen, besonders bevorzugt um mindestens 3 Hazen, ganz besonders bevorzugt um mindestens 5 Hazen und insbesondere um mindestens 10 Hazen. Es kann wünschenswert sein, die Farbzahl um mindestens 15 Hazen oder sogar um mindestens 20 Hazen zu verringern.

Es werden Strahlungsdosen von 20-40000 J/cm² eingesetzt. Höhere Dosen können beispielsweise dann sinnvoll sein, wenn das Produkt stark gefärbt ist.

Als Strahlungsquellen sind z.B. Quecksilber-Niederdruckstrahler, -Mitteldruckstrahler mit Hochdruckstrahler geeignet, die gegebenenfalls undotiert, Gallium- oder Eisendotiert sein können, sowie Leuchtstoffröhren, Impulsstrahler, Metallhalogenidstrahler, Excimerstrahler, Laser, LED, gepulste Lampen (Blitzlicht) oder Halogenlampen.

Selbstverständlich sind auch mehrere gleichartige oder unterschiedliche Strahlungsquellen einsetzbar, um die gewünschte Energiedosis oder Spektralverteilung zu erzielen.
Diese können auch in jeweils unterschiedlichen Wellenlängebereichen strahlen.

Es ist auch möglich, durch geeignete optische Filter spezielle Wellenlängenbereiche aus dem Bestrahlungsspektrum auszublenden, um unerwünschte Photoreaktionen zu vermeiden.

Möglich ist ebenfalls, das Glas zwischen Strahlungsquelle und zu bestrahlendem Produkt so zu wählen, daß unerwünschte Lichtwellenlängen der Strahlungsquelle absorbiert werden. Beispielsweise kann das relativ teure Quarzglas durch Borsilikatglas je nach Strahlungsquelle ersetzt werden, um produktschädigende kurzwellige Strahlung auszufiltern.

Es können optische Filter eingesetzt werden, die z.B. sehr kurzwellige Stahlung, bevorzugt unter 250 nm sperren oder Filter die beispielsweise sowohl kurzwellige UV- als auch langwellige IR-Strahlung (= Wärmestrahlung), insbesondere der Wellenlängen von 780 nm bis 1 mm absperren.

Das Polyisocyanat kann erfindungsgemäß über eine Dauer von 5 Minuten bis zu 600 Stunden, bevorzugt von 20 min bis 8 Stunden bestrahlt werden.

Die Temperatur des Polyisocyanat bei der Bestrahlung spielt dabei lediglich eine untergeordnete Rolle. Die untere Temperaturgrenze wird dadurch festgelegt, daß das Polyisocyanat pumpfähig sein sollte, die obere Grenze wird durch dessen Temperaturstabilität festgelegt. Bevorzugt beträgt die Temperatur von Umgebungstemperatur bis 130 °C, besonders bevorzugt von 20 °C bis 100 °C, ganz besonders bevorzugt von 30 °C bis 80 °C und insbesondere von 30 °C bis 70 °C.

Die Bestrahlung kann erfindungsgemäß kontinuierlich oder diskontinuierlich erfolgen. Dabei kann das Polyisocyanat ruhen oder bevorzugt bewegt werden, beispielsweise durch Umpumpen oder Rühren.

In einer bevorzugten Ausführungsform wird die Strahlungsquelle, oder mehrere, falls gewünscht oder erforderlich, in einen Vorratsbehälter, beispielsweise einen Lagertank, mit Polyisocyanat getaucht und das Polyisocyanat bewegt.

In einer weiteren bevorzugten Ausführungsform wird die Strahlungsquelle, oder mehrere Strahlungsquellen, falls gewünscht oder erforderlich, in einen Vorratsbehälter, beispielsweise einen Lagertank, in oder über dem Polyisocyanat positioniert und das Polyisocyanat bewegt.

In einer weiteren bevorzugten Ausführungsform wird das Polyisocyanat durch ein Rohrstück geleitet, das für die gewünschte Wellenlänge des eingestrahlten Lichtes zumindest teilweise durchlässig ist, beispielsweise aus Quartzglas gefertigt, und das Polyisocyanat bestrahlt. Anordnungen für derartige Vorrichtungen sind prinzipiell aus der Trinkwasseraufbereitung bekannt und können in für Chemieanlagen typischer Weise an die Anforderungen angepaßt werden.

Denkbar ist auch, daß die Bestrahlung in einer Apparatur bereits während der Herstellung erfolgt, beispielsweise in der Abtrennung von unumgesetzten monomeren Isocyanat (siehe oben), indem beispielsweise ein Fallfilmverdampfer mit einem Quartzfenster versehen wird oder eine Strahlungsquelle innerhalb des Fallfilmverdampfers plaziert wird.

Zur Verringerung der Viskosität kann es erforderlich sein das Polyisocyanat mit einem Lösungsmittel zu versetzen.

Beispiele für derartige Lösungsmittel sind aromatische und/oder (cyclo)aliphatische Kohlenwasserstoffe und deren Gemische, halogenierte Kohlenwasserstoffe, Ketone, Ester und Ether.

Bevorzugt sind aromatische Kohlenwasserstoffe, (cyclo)aliphatische Kohlenwasserstoffe, Alkansäurealkylester, alkoxylierte Alkansäurealkylester und deren Gemische.

Besonders bevorzugt sind ein- oder mehrfach alkylierte Benzole und Naphthaline, Alkansäurealkylester und alkoxylierte Alkansäurealkylester sowie deren Gemische.

Als aromatische Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), Solvesso® 150 (Siedebereich etwa 182 - 207 °C) und Solvesso® 200 (CAS-Nr. 64742-94-5), sowie die Shellsol®-Marken der Firma Shell, Caromax® (z.B. Caromax® 18) der Firma Petrochem Carless und Hydrosol der Firma DHC (z.B. als Hydrosol® A 170). Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

Der Gehalt an aliphatischen Kohlenwasserstoffen beträgt in der Regel weniger als 5, bevorzugt weniger als 2,5 und besonders bevorzugt weniger als 1 Gew%.

Halogenierte Kohlenwasserstoffe sind beispielsweise Chlorbenzol und Dichlorbenzol oder dessen Isomerengemische sowie Parachlorbenzotrifluorid (Oxsol® 100).

Ester sind beispielsweise n-Butylacetat, Ethylacetat, 1-Methoxypropylacetat-2 und 2-Methoxyethylacetat.

Ether sind beispielsweise THF, Dioxan sowie die Dimethyl-, -ethyl- oder -n-butylether von Ethylenglykol, Diethylenglykol, Triethylenglykol, Propylenglykol, Dipropylenglykol oder Tripropylenglykol.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen, beispielsweise Petrolether oder Ligroin.

Weiterhin bevorzugt sind n-Butylacetat, Ethylacetat, 1-Methoxypropylacetat-2, 2-Methoxyethylacetat, tert.-Butylacetat, sowie deren Gemische, insbesondere mit den oben aufgeführten aromatischen Kohlenwasserstoffgemischen.

Weiterhin bevorzugt sind Ketone, beispielsweise Aceton, Methylethylketon und Methylamylketon.

Derartige Gemische können im Volumenverhältnis 5:1 bis 1:5 erstellt werden, bevorzugt im Volumenverhältnis 4:1 bis 1:4, besonders bevorzugt im Volumenverhältnis 3:1 bis 1:3 und ganz besonders bevorzugt im Volumenverhältnis 2:1 bis 1:2.

Bevorzugte Beispiele sind Butylacetat/Xylol, Methoxypropylacetat/Xylol 1:1, Butylacetat/Solventnaphtha 100 1:1, Butylacetat/Solvesso® 100 1:2 und Kristallöl 30/Shellsol® A 3:1.

Durch das erfindungsgemäße Verfahren können solche Polyisocyanate, die eine Farbe aufweisen, wieder entfärbt werden und weisen anschließend auch während einer Lagerung eine gute Farbstabilität auf. Infolgedessen eignen sich die entsprechend behandelten Polyisocyanate gut zur Verwendung in Lacken:
Die erfindungsgemäß erhaltenen Polyisocyanate können verwendet werden zur Herstellung von Polyurethanen und Polyurethanlacken, beispielsweise für einkomponentige, zweikomponentige, strahlungshärtbare oder Pulverlackbeschichtungssysteme sowie damit hergestellt Lacke zur Beschichtung von verschiedenen Substraten, wie z. B. Holz, Holzfurnier, Papier, Pappe, Karton, Textil, Leder, Vlies, Kunststoffoberflächen, Glas, Keramik, mineralische Baustoffe, Metalle oder beschichtete Metalle.

Bei einer Verwendung in Beschichtungsmitteln können die erfindungsgemäßen Polyisocyanate insbesondere in Grundierungen, Füllern, pigmentierten Decklacken, Basislacken, Klarlacken und Topcoat im Bereich Autoreparatur- oder Großfahrzeuglackierung eingesetzt werden. Besonders geeignet sind solche Beschichtungsmittel für Anwendungen, in denen eine besonders hohe Applikationssicherheit, Außenwitterungsbeständigkeit, Optik, Lösemittel-, Chemikalien- und Wasserfestigkeit gefordert werden, wie in der Autoreparatur- und Großfahrzeuglackierung sowie bei Nutzfahrzeugen im landwirtschaftlichen und Baubereich.

Derartige Beschichtungsmassen eignen sich als oder in Außenbeschichtungen, also solche Anwendungen, die dem Tageslicht ausgesetzt sind, bevorzugt von Gebäudeteilen, Innenbeschichtungen, Beschichtungen auf (Groß-)Fahrzeugen und Flugzeugen und industriellen Anwendungen, Brücken, Gebäuden, Strommasten, Tanks, Containern, Pipelines, Kraftwerken, chemischen Anlagen, Schiffen, Kränen, Pfählen, Spundwänden, Armaturen, Rohren, Fittings, Flanschen, Kupplungen, Hallen, Dächern und Baustahl. Insbesondere werden die erfindungsgemäßen Beschichtungsmassen als oder in Automobilklar- und -decklacke(n) eingesetzt. Weitere bevorzugte Einsatzgebiete sind Can-Coating und Coil-Coating.

Besonders eignen sie sich als Grundierungen, Füller, pigmentierte Decklacke und Klarlacke im Bereich Industrie-, Holz-, Auto-, insbesondere OEM- Lackierung, oder Dekolackierung eingesetzt werden. Ganz besonders geeignet sind die Beschichtungsmittel für Anwendungen, in denen eine besonders hohe Applikationssicherheit, Außenwitterungsbeständigkeit, Optik, Kratzbeständigkeit, Lösemittel- und/oder Chemikalienbeständigkeit gefordert werden. Durch ihre niedrige Farbzahl und hohe Farbstabilität sind sie insbesondere für Beschichtungsmassen für Klarlacke interessant. Sie sind insbesondere interessant in Kombination mit Antioxidantien und Lewis-sauren Verbindungen, gegebenenfalls in Anwesenheit von Lösungsmittel, insbesondere bei Lagerung.

### Beispiele

Die Farbzahlmessung in dieser Schrift erfolgt in Hazen (Hz) nach DIN EN 1557 auf einem Lico 300-Gerät der Firma Lange in einer 5 cm Messkuvette mit einem Volumen von 5 mL.

### Beispiel 1

600 g Isophorondiisocyanat (IPDI), hergestellt durch einen Phosgenprozess, wurden bei Raumtemperatur vorgelegt, unter Vakuum 1 h gehalten und anschließend auf 50°C erhitzt.

Es erfolgte die Zugabe von 160 ppm N,N,N-Trimethyl-N-benzylammoniumhydroxid in Form einer 3 %igen Lösung in Ethylhexanol. Die Temperatur stieg von 50°C auf 61°C an. Anschließend wurde auf 65°C aufgeheizt. Der NCO-Wert erreichte nach einer Nachreaktionszeit von 100 min 32,2%.

Durch schnelles Erhitzen auf 140°C wurde die Reaktion gestoppt. Nach der Filtration wurde das überschüssige IPDI in einem Dünnschichtverdampfer bei 195°C Heizmediumtemperatur fast vollständig entfernt. Das Produkt wurde 70%ig in Butylacetat gelöst. Man erhält eine gelblich gefärbte Produktlösung (Farbzahl 68 Hz) mit einem NCO-Gehalt von 12,4%.

### Beispiel 2

Die Verarbeitung erfolgte analog Beispiel 1, jedoch wurde nach Erreichen eines NCO-Wertes von 32% die Reaktion mit einer molaren Menge von Butylcarbamat, bezogen auf den eingesetzten Katalysator, bei 65 °C abgebrochen.
Farbzahl : 39 Hz

### Beispiel 3

Die Verarbeitung erfolgte analog Beispiel 1, jedoch wurde als Trimerisierungskatalysator DABCO® TMR verwendet. Nach Erreichen eines NCO-Wertes von 32,1 % wurde die Reaktion mit einer molaren Menge von XHC-20 (2-Hydroxyethylcarbamat, Fa. Huntsman), bezogen auf den eingesetzten Katalysator, bei 65 °C abgebrochen. Farbzahl: 35 Hz

### Beispiel 4

Der Versuch gemäß Beispiel 3 wurde mit IPDI, hergestellt mittels einem Harnstoffverfahren, wiederholt.
Farbzahl: 42 Hz

### Beispiel 5

Der Versuch gemäß Beispiel 3 wurde mit IPDI aus einem Harnstoffverfahren wiederholt. Die Reaktion wurde jedoch thermisch bei 140°C gestoppt Farbzahl: 55 Hz

### Beispiel 6

Der Versuch gemäß Beispiel 4 wurde mit IPDI aus einem Phosgenverfahren wiederholt.

Die Reaktion wurde bei einem NCO-Wert von 32,5 % thermisch bei 140°C gestoppt Farbzahl : 88 Hz

### Beispiel 7

600 g frisch destilliertes IPDI aus einem Phosgenprozess wurden mit 6 g Methanol versetzt und 2 Stunden bei 90°C gerührt. Die Temperatur wurde dann auf 65°C abgesenkt. Dann erfolgte die Zugabe 150 ppm DABCO® TMR. Bei einem NCO-Wert von 31,7% erfolgte Zugabe von äquimolarer Menge Butylcarbamat, bezogen auf den eingesetzten Katalysator.

Anschließend erfolgte die destillative Aufarbeitung bei 190°C/ 1mbar Farbzahl : 41 Hz

### Beispiel 8

600 g frisch destilliertes IPDI aus einem Harnstoffprozess wurden mit 6 g Ethanol versetzt und 2 Stunden bei 90°C gerührt. Die Temperatur wurde dann auf 65°C abgesenkt. Dann erfolgte die Zugabe 160 ppm DABCO® TMR. Bei einem NCO-Wert von 31,7% erfolgte Zugabe von äquimolarer Menge XHC-20 (Fa. Huntsman), bezogen auf den eingesetzten Katalysator.

Weiterverarbeitung analog wie unter Beispiel 1 beschrieben.
Farbzahl : 35 Hz

### Beispiel 9

600 g einer Mischung aus 50% IPDI aus einem Phosgen-Prozess und 50% aus einem Harnstoffprozess wurden mit 8 g 2-Ethylhexanol versetzt und 2 Stunden bei 115°C gerührt. Die Temperatur wurde dann auf 65°C abgesenkt. Dann erfolgte die Zugabe 185 ppm DABCO® TMR gelöst in 2-Ethyl-1,3-hexandiol (2 %ige Lösung) . Bei einem NCO-Wert von 31,7% erfolgte Zugabe von 2facher äquimolarer Menge tert.-Butylcarbamat, bezogen auf den eingesetzten Katalysator.

Weiterverarbeitung analog wie unter Beispiel 1 beschrieben.
Farbzahl : 37 Hz

### Beispiel 10

600 g einer Mischung aus 50% IPDI aus einem Phosgen-Prozess und 50% aus einem Harnstoffprozess wurden mit 8 g 2-Ethylhexanol versetzt und 2 Stunden bei 115°C gerührt. Die Temperatur wurde dann auf 65°C abgesenkt. Dann erfolgte die Zugabe 185 ppm DABCO® TMR gelöst in 2-Ethyl-1,3-hexandiol (2 %ige Lösung) . Bei einem NCO-Wert von 32.7% erfolgte die thermische Stoppung bei 140°C Weiterverarbeitung analog wie unter Beispiel 1 beschrieben.
Farbzahl : 85 Hz

### Beispiel 11

588 g 1,6-Hexamethylendiisocyanat (HDI) aus einem Phosgenprozess wurden bei Raumtemperatur vorgelegt, unter Stickstoff 1 h gehalten und anschließend auf 40°C erhitzt. Es erfolgte die Zugabe von 60 ppm N,N,N-Trimethyl-N-benzylammonium hydroxid in Form einer 3 %igen Lösung in Ethylhexanol.

Die Temperatur stieg von 40°C auf 52°C an. Anschließend wurde auf 60°C aufgeheizt. Der NCO-Wert erreichte nach einer Nachreaktionszeit von 100 min 40,6%.
Durch schnelles Erhitzen auf 130°C wurde die Reaktion gestoppt. Nach der Filtration wurde das überschüssige HDI in einem Dünnschichtverdampfer bei 170°C Außentemperatur fast vollständig entfernt.
Man erhält ein gelblich befärbtes Produkt (Farbzahl 78 Hz) mit einem NCO-Gehalt von 21,7% und einer Viskosität von 2400 mPas.

### Beispiel 12

Die Verarbeitung erfolgte analog Beispiel 1, jedoch wurde als Trimerisierungskatalysator DABCO® TMR verwendet. Nach Erreichen eines NCO-Wertes von 40,6 % wurde die Reaktion mit einer molaren Menge von XHC-20 (Fa. Huntsman, bezogen auf den eingesetzten Katalysator, bei 65 °C abgebrochen.
Farbzahl nach Destillation : 65 Hz

### Beispiel 13

588 g frisch destilliertes HDI aus einem Harnstoffverfahren wurden mit 12 g Methanol versetzt und 2 Stunden bei 80°C gerührt. Die Temperatur wurde dann auf 65°C abgesenkt. Dann erfolgte die Zugabe 150 ppm DABCO® TMR. Bei einem NCO-Wert von 38,7% erfolgte Zugabe von äquimolarer Mange Butylcarbamat, bezogen auf den eingesetzten Katalysator.
Es wurde 1 Stunde nachgerührt und erneut der NCO-Wert gemessen.
NCO-Wert nach der Nachreaktion : 38,6%
Anschließend erfolgte die destillative Aufarbeitung bei 170°C/ 1mbar
Farbzahl nach Destillation : 48 Hz

### Beispiel 14

588 g frisch destilliertes HDI aus einem Phosgenprozess wurden mit 12 g Ethylhexanol versetzt und 2 Stunden bei 115°C gerührt. Die Temperatur wurde dann auf 65°C abgesenkt. Dann erfolgte die Zugabe 24 ppm N,N,N-Trimethyl-N-benzylammonium hydroxid, gelöst in 2-Ethyl-1,3-hexandiol (0,5%ige Lösung) . Bei einem NCO-Wert von 38,7% erfolgte Zugabe von zweifacher äquimolarer Menge tert.-Butylcarbamat, bezogen auf den eingesetzten Katalysator.
Es wurde 1 Stunde nachgerührt und erneut der NCO-Wert gemessen.
NCO-Wert nach der Nachreaktion : 38,7%.
Weiterverarbeitung wie unter Beispiel 7 beschrieben.
Farbzahl nach Destillation : 58 Hz

### Beispiel 15

588 g frisch destillierte HDI-Mischung aus dem Phosgenprozess und Harnstoffprozess (1:1) wurden mit 12 g Ethylhexanol versetzt und 2 Stunden bei 115°C gerührt. Die Temperatur wurde dann auf 65°C abgesenkt. Dann erfolgte die Zugabe 24 ppm DABCO® TMR in 2-Ethyl-1,3-hexandiol (0,5%ige Lösung). Bei einem NCO-Wert von 38,7% erfolgte Zugabe äquimolarer Menge tert.-Butylcarbamat, bezogen auf den eingesetzten Katalysator.
Es wurde 1 Stunde nachgerührt und erneut der NCO-Wert gemessen.
NCO-Wert nach der Nachreaktion : 38,7%.
Weiterverarbeitung wie unter Beispiel 7 beschrieben.
Farbzahl nach Destillation : 51 Hz

### Ergebnisse nach der Bestrahlung

Die verschiedenen Muster wurden in einem Panacol-Elosol-Bestrahlungsgerät mit folgenden UV-Lampen 4 Stunden bestrahlt :

| Lampentyp | Energie [mW/cm2] |
|---|---|
| ES 450 | 17,2 |
| ES 460 | 5,6 |
| ES 465 | 14,1 |
| ES 470 | 8,6 |

Als Energiemessgerät wurde ein UV-Meter (Sonde Nr. 724 (UV-A Bereich)) von der Fa. Hönle verwendet.

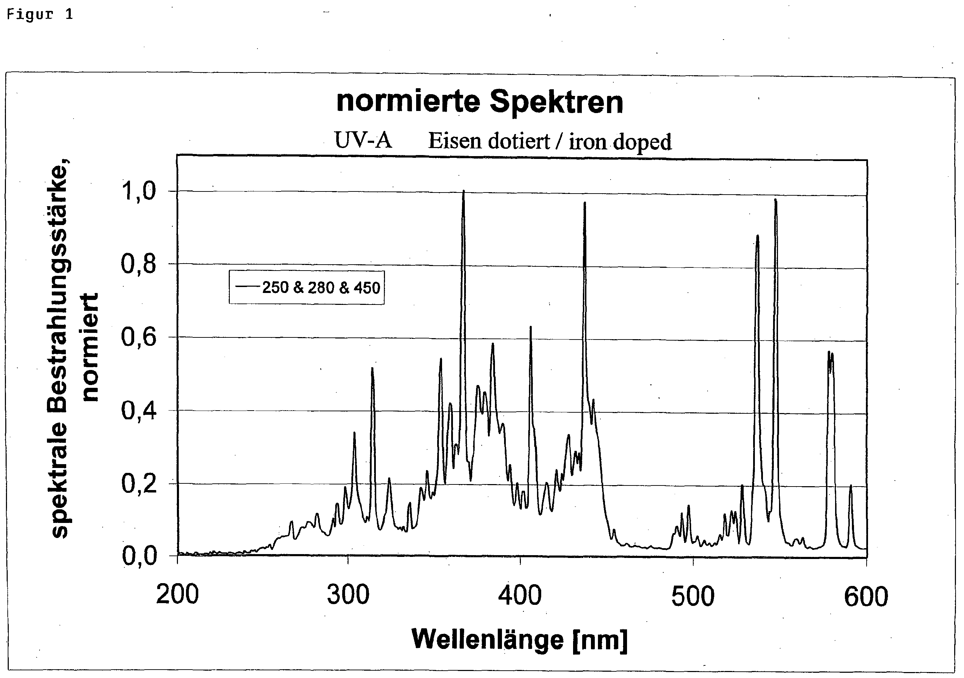

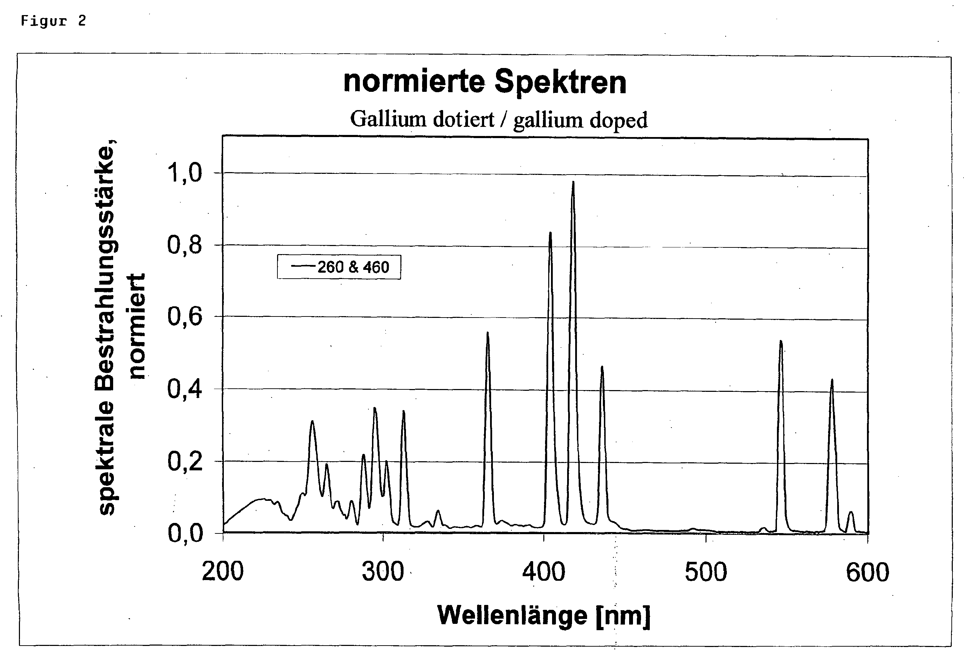

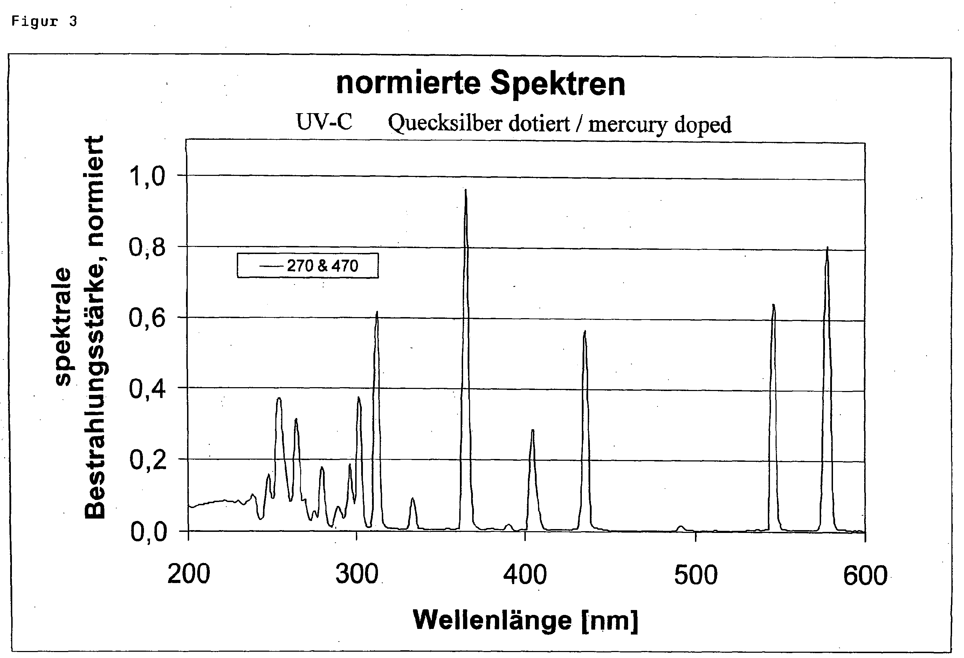

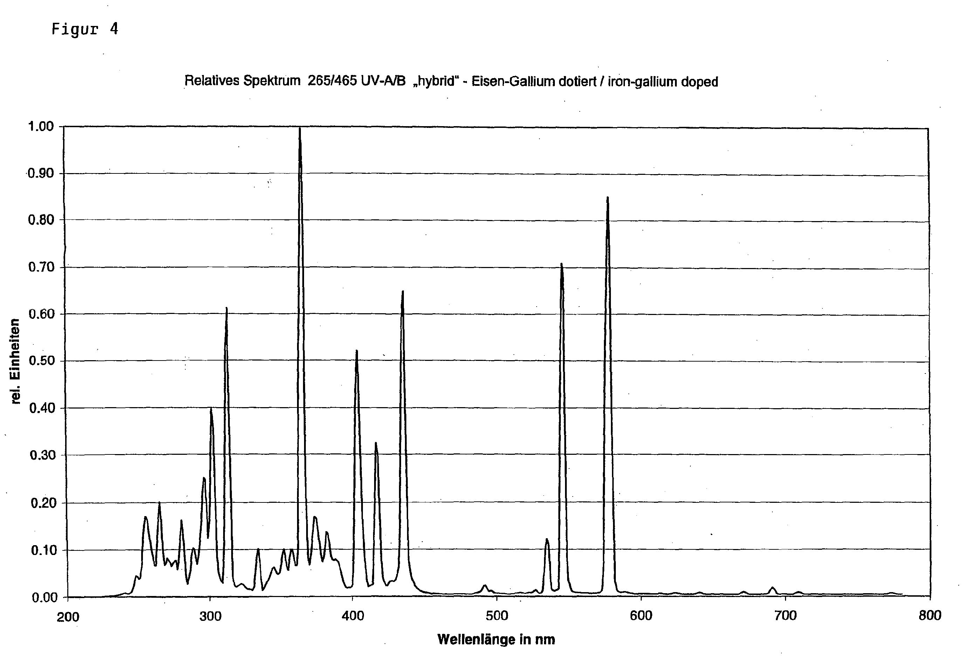

Die Spektren der Lampen sind in den Figuren 1 bis 4 dargestellt:
- Figur 1:: ES 450
- Figur 2:: ES 460
- Figur 3:: ES 470
- Figur 4:: ES 465

Die Beispiele sind in Tabelle 1 dargestellt.

**Tabelle 1**

| Beispiel | Farbzahl [Hz] vor Bestrahlung | Farbzahl [Hz] nach Bestrahlung | | | |
|---|---|---|---|---|---|
| | | ES 450 | ES 460 | ES 465 | ES 470 |
| 1 | 68 | 27 | 32 | 35 | 37 |
| 2 | 39 | 19 | 21 | 24 | 21 |
| 3 | 35 | 21 | 24 | 23 | 25 |
| 4 | 42 | 25 | 26 | 27 | 26 |
| 5 | 55 | 31 | 32 | 35 | 32 |
| 6 | 88 | 34 | 45 | 38 | 46 |
| 7 | 41 | 23 | 26 | 24 | 27 |
| 8 | 35 | 20 | 28 | 24 | 31 |
| 9 | 37 | 21 | 29 | 24 | 29 |
| 10 | 85 | 31 | 42 | 34 | 45 |
| 11 | 78 | 23 | 36 | 31 | 38 |
| 12 | 65 | 25 | 32 | 26 | 32 |
| 13 | 48 | 31 | 35 | 32 | 35 |
| 14 | 58 | 32 | 35 | 33 | 37 |
| 15 | 51 | 29 | 33 | 30 | 35 |

Es zeigte sich, dass alle Lampentypen die Produktfarbe reduzieren konnten.

Die Proben wurden nach der UV-Behandlung über 9 Wochen (Wo) bei 50°C gelagert.

Die Beispiele sind in Tabelle 2 dargestellt.

**Tabelle 2**

| Beispiel | unbestrahlt | | ES 450 | | ES 460 | | ES 465 | | ES 470 | |
|---|---|---|---|---|---|---|---|---|---|---|
| | 0 Wo | 9 Wo | 0 Wo | 9 Wo | 0 Wo | 9 Wo | 0 Wo | 9 Wo | 0 Wo | 9 Wo |
| 1 | 68 | 135 | 27 | 33 | 32 | 35 | 35 | 34 | 37 | 65 |
| 2 | 39 | 82 | 19 | 25 | 21 | 28 | 24 | 26 | 21 | 45 |
| 3 | 35 | 79 | 21 | 26 | 24 | 31 | 23 | 29 | 25 | 35 |
| 4 | 42 | 92 | 25 | 31 | 26 | 35 | 27 | 31 | 26 | 47 |
| 5 | 55 | 105 | 31 | 37 | 32 | 39 | 35 | 38 | 32 | 54 |
| 6 | 88 | 145 | 34 | 41 | 45 | 44 | 38 | 49 | 46 | 52 |
| 7 | 41 | 79 | 23 | 31 | 26 | 36 | 24 | 35 | 27 | 54 |
| 8 | 35 | 67 | 20 | 27 | 28 | 31 | 24 | 32 | 31 | 42 |
| 9 | 37 | 69 | 21 | 33 | 29 | 41 | 24 | 33 | 29 | 37 |
| 10 | 85 | 134 | 31 | 41 | 42 | 42 | 34 | 51 | 45 | 58 |
| 11 | 78 | 156 | 23 | 31 | 36 | 45 | 31 | 47 | 38 | 42 |
| 12 | 65 | 132 | 25 | 35 | 32 | 48 | 26 | 41 | 32 | 53 |
| 13 | 48 | 98 | 31 | 55 | 35 | 62 | 32 | 47 | 35 | 61 |
| 14 | 58 | 78 | 32 | 43 | 35 | 49 | 33 | 41 | 37 | 57 |
| 15 | 51 | 68 | 29 | 41 | 33 | 51 | 30 | 39 | 35 | 43 |

Bei allen Proben zeigte sich ein deutlich verbesserte Lagerstabilität der Produkteigenschaften.

### Beispiel 16 :

Zur weiteren Prüfung der Lagerstabilität wurden die hergestellten Polyisocyanate in einer Formulierung, enthaltend das Polyisocyanat, einen Lewis-sauren Katalysator und organische Lösungsmittel bei erhöhter Temperatur geprüft.
Für diese Prüfung wurden 50 Gew.% Polyisocyanat mit 50 Gew.% Lösungsmittel, bestehend aus 75% Solventnaphtha und 25% Butylacetat, welches 1000 Gew.ppm Dibutyldizinnlaurat (bezogen auf die Gesamtmenge an Lösungsmittel) enthielt, gegeben. Nach der Durchmischung der Formulierung bei Raumtemperatur wurde die Mischung bei 50°C unter Stickstoffatmosphäre gelagert.
Nach 1, 2, 4 und 8 Wochen wurde der Farbanstieg gemessen.

**Lagerung bei 50°C**

| Muster | Bestrahlung mit ES 450 | Farbe nach Formulierung | 1 Wochen | 2 Wochen | 4 Wochen | 8 Wochen |
|---|---|---|---|---|---|---|
| 1 | nein | 45 | 71 | 118 | 165 | 298 |
| | ja | 16 | 19 | 65 | 112 | 240 |
| 2 | nein | 33 | 51 | 88 | 112 | 240 |
| | ja | 15 | 18 | 34 | 97 | 211 |
| 4 | nein | 32 | 46 | 81 | 123 | 231 |
| | ja | 16 | 18 | 38 | 102 | 179 |
| 5 | nein | 41 | 63 | 112 | 157 | 289 |
| | ja | 25 | 27 | 67 | 109 | 245 |
| 7 | nein | 29 | 41 | 82 | 110 | 213 |
| | ja | 14 | 17 | 34 | 94 | 167 |
| 8 | nein | 27 | 37 | 75 | 93 | 191 |
| | ja | 12 | 15 | 46 | 78 | 154 |
| 10 | nein | 61 | 93 | 165 | 245 | 398 |
| | ja | 23 | 31 | 113 | 187 | 365 |
| 11 | nein | 58 | 89 | 161 | 231 | 393 |
| | ja | 18 | 20 | 113 | 189 | 312 |
| 12 | nein | 51 | 81 | 141 | 197 | 347 |
| | ja | 21 | 24 | 87 | 150 | 310 |
| 13 | nein | 37 | 57 | 111 | 160 | 261 |
| | ja | 22 | 29 | 79 | 140 | 235 |

### Beispiel 17 :

Weiterhin wurde die Viskosität der gemäß Beispiel 16 hergestellten Muster mit einem Physica Rheolab MC1 nach DIN 5301 und einer Schergeschwindigkeit von 250 s⁻¹ gemessen.

**Lagerung bei 50°C**

| Muster | Bestrahlung | Viskosität nach Formulierung | 1 Wochen | 2 Wochen | 4 Wochen | 8 Wochen |
|---|---|---|---|---|---|---|
| 1 | nein | 970 | 1110 | 1271 | 1631 | 1940 |
| | ja | 972 | 995 | 1170 | 1506 | 1754 |
| 2 | nein | 1130 | 1305 | 1561 | 2046 | 2260 |
| | ja | 1170 | 1300 | 1527 | 1964 | 2146 |
| 4 | nein | 1030 | 1170 | 1372 | 1844 | 2060 |
| | ja | 1035 | 1179 | 1419 | 1761 | 2016 |
| 5 | nein | 1231 | 1510 | 1760 | 2296 | 2610 |
| | ja | 1250 | 1441 | 1701 | 2213 | 2310 |
| 7 | nein | 960 | 1109 | 1270 | 1651 | 2140 |
| | ja | 965 | 1090 | 1283 | 1630 | 1930 |
| 8 | nein | 920 | 1070 | 1231 | 1631 | 1951 |
| | ja | 923 | 1051 | 1206 | 1551 | 1753 |
| 10 | nein | 1031 | 1189 | 1381 | 1849 | 2317 |
| | ja | 1040 | 1156 | 1302 | 1691 | 2080 |
| 11 | nein | 530 | 613 | 701 | 917 | 1121 |
| | ja | 542 | 612 | 695 | 879 | 1084 |
| 12 | nein | 578 | 661 | 806 | 1067 | 1239 |
| | ja | 602 | 625 | 751 | 974 | 1204 |
| 13 | nein | 510 | 597 | 732 | 953 | 1160 |
| | ja | 519 | 586 | 681 | 889 | 1038 |

Es zeigte sich, dass die UV-bestrahlten Proben sowohl bei den Farbwerten, als auch bei den Viskositäten bessere Ergebnisse erzielten.

## Patentansprüche

1. Verfahren zur Herstellung farbreduzierter (cyclo)aliphatischer Polyisocyanate, **dadurch gekennzeichnet, daß** man das Polyisocyanat auf Basis (cyclo)aliphatischer Diisocyanate mit höherer als gewünschter Farbzahl mit Licht der Wellenlänge von 200 bis 600 nm mit einer Strahlungsdose von 20 bis 40000 J/cm² in diesem Wellenlängenbereich bestrahlt, die ausreicht um die Farbzahl um mindestens 1 Hazen zu verringern, wobei das man Verfahren ohne Zusatz von Ozon, Sauerstoff oder ozonhaltigen Gasgemischen, organischen Persäuren Peroxid oder Hydroperoxid durchführt und wobei die Farbzahl nach DIN EN 1557 bestimmt wird.

2. Verfahren gemäß Anspruch 1, **dadurch gekennzeichnet, daß** es sich bei den Polyisocyanaten um Polyisocyanate auf Basis Isophorondiisocyanat und/oder 1,6-Hexamethylendiisocyanat handelt.

3. Verfahren gemäß einem der vorstehenden Ansprüche, **dadurch gekennzeichnet, daß** das Polyisocyanat Isocyanurat-, Urethan- und/oder Allophanatgruppen aufweist.

4. Verfahren gemäß einem der vorstehenden Ansprüche, **dadurch gekennzeichnet, daß** das Polyisocyanat erhalten worden ist durch Umsetzung von monomeren Isocyanaten durch Einwirkung mindestens eines Katalysators, der mindestens ein quartäres Stickstoffatom aufweist.

5. Verfahren gemäß Anspruch 4, **dadurch gekennzeichnet, daß** es sich bei dem Katalysator um eine Verbindung der Formel
R¹R²R³R⁴N⁺ Y⁻
handelt, worin
R¹, R², R³ und R⁴ jeweils unabhängig voneinander gegebenenfalls durch Aryl, Alkyl, Aryloxy, Alkyloxy, Heteroatome und/oder Heterocyclen substituiertes C₁-C₂₀-Alkyl, C₆ - C₁₂-Aryl oder C₅ - C₁₂-Cycloalkyl und Y⁻ ein beliebiges Anion bedeuten.

6. Verfahren gemäß Anspruch 5, **dadurch gekennzeichnet, daß** R¹, R², R³ und R⁴ jeweils unabhängig voneinander ausgewählt sind aus der Gruppe bestehend aus Methyl, Ethyl, n-Butyl, Octyl, Benzyl, 2-Hydroxyethyl und 2-Hydroxypropyl.

7. Verfahren gemäß Anspruch 5 oder 6, **dadurch gekennzeichnet, daß** das Anion Y⁻ ausgewählt aus der Gruppe bestehend aus Fluorid, Hydroxid, Carboxylat, Carbonat und α-Hydroxycarboxylat.

8. Verfahren gemäß Anspruch 5, **dadurch gekennzeichnet, daß** das Anion Y⁻ ausgewählt aus der Gruppe bestehend aus Formiat, Acetat, Propionat und 2-Ethylhexanoat.

9. Verwendung von Polyisocyanaten, erhalten nach einem Verfahren gemäß einem der vorstehenden Ansprüche, in Beschichtungsmitteln, Grundierungen, Füllern, pigmentierten Decklacken, Basislacken und Klarlacken im Bereich Autoreparatur- oder Großfahrzeuglackierung, in der Autoreparatur- und Großfahrzeuglackierung sowie bei Nutzfahrzeugen im landwirtschaftlichen und Baubereich.

## Claims

1. A process for preparing color-reduced (cyclo)aliphatic polyisocyanates, wherein the polyisocyanate which is based on (cyclo)aliphatic diisocyanates and has a higher than desired color number is irradiated with light having a wavelength of from 200 to 600 nm at a radiation dose of from 20 to 40 000 J/cm² in this wavelength range which is sufficient to reduce the color number by at least 1 Hazen, where the process is carried out without addition of ozone, oxygen or ozone-containing gas mixtures, organic peracids, peroxide or hydroperoxide and where the color number is determined according to DIN EN 1557.

2. The process according to claim 1, wherein the polyisocyanates are polyisocyanates based on isophorone diisocyanate and/or hexamethylene 1,6-diisocyanate.

3. The process according to either of the preceding claims, wherein the polyisocyanate has isocyanurate, urethane and/or allophanate groups.

4. The process according to any of the preceding claims, wherein the polyisocyanate has been obtained by reaction of monomeric isocyanates in the presence of at least one catalyst having at least one quaternary nitrogen atom.

5. The process according to claim 4, wherein the catalyst is a compound of the formula
R¹R²R³R⁴N⁺ Y⁻
where
R¹, R², R³ and R⁴ are each, independently of one another, optionally aryl-, alkyl-, aryloxy-, alkyloxy-, heteroatom- and/or heterocycle-substituted C₁ - C₂₀-alkyl, C₆ - C₁₂-aryl or C₅-C₁₂-cycloalkyl and Y⁻ is any anion.

6. The process according to claim 5, wherein R¹, R², R³ and R⁴ are selected independently from the group consisting of methyl, ethyl, n-butyl, octyl, benzyl, 2-hydroxyethyl and 2-hydroxypropyl.

7. The process according to claim 5 or 6, wherein the anion Y⁻ is selected from the group consisting of fluoride, hydroxide, carboxylate, carbonate and α-hydroxycarboxylate.

8. The process according to claim 5, wherein the anion Y⁻ is selected from the group consisting of formate, acetate, propionate and 2-ethylhexanoate.

9. The use of polyisocyanates obtained by a process according to any of the preceding claims in coating compositions, primers, fillers, pigmented topcoats, undercoats and clear coatings in the field of automobile repair coating or large vehicle coating, in automobile repair coating and large vehicle coating and also in commercial vehicles in the agricultural and building sectors.

## Revendications

1. Procédé de fabrication de polyisocyanates (cyclo)aliphatiques de couleur réduite, **caractérisé en ce que** le polyisocyanate à base de diisocyanates (cyclo)aliphatiques ayant un indice de couleur plus élevé que souhaité est exposé à une lumière d'une longueur d'onde de 200 à 600 nm à une dose de rayonnement de 20 à 40 000 J/cm² dans la plage de longueur d'onde qui est suffisante pour réduire l'indice de couleur d'au moins 1 unité Hazen, le procédé étant réalisé sans ajout d'ozone, d'oxygène ou de mélanges gazeux contenant de l'ozone, de peracides organiques, de peroxyde ou d'hydroperoxyde, et l'indice de couleur étant déterminé selon DIN EN 1557.

2. Procédé selon la revendication 1, **caractérisé en ce que** les polyisocyanates sont des polyisocyanates à base de diisocyanate d'isophorone et/ou de diisocyanate de 1,6-hexaméthylène.

3. Procédé selon l'une quelconque des revendications précédentes, **caractérisé en ce que** le polyisocyanate comprend des groupes isocyanurate, uréthane et/ou allophanate.

4. Procédé selon l'une quelconque des revendications précédentes, **caractérisé en ce que** le polyisocyanate a été obtenu par mise en réaction d'isocyanates monomères sous l'effet d'au moins un catalyseur, qui comprend au moins un atome d'azote quaternaire.

5. Procédé selon la revendication 4, **caractérisé en ce que** le catalyseur est un composé de formule
R¹R²R³R⁴N⁺ Y⁻
dans laquelle
R¹, R², R³ et R⁴ signifient chacun indépendamment les uns des autres alkyle en C₁-C₂₀, aryle en C₆-C₁₂ ou cycloalkyle en C₅-C₁₂, éventuellement substitué par aryle, alkyle, aryloxy, alkyloxy, des hétéroatomes et/ou des hétérocycles, et Y⁻ signifie un anion quelconque.

6. Procédé selon la revendication 5, **caractérisé en ce que** R¹, R², R³ et R⁴ sont chacun choisis indépendamment les uns des autres dans le groupe constitué par méthyle, éthyle, n-butyle, octyle, benzyle, 2-hydroxyéthyle et 2-hydroxypropyle.

7. Procédé selon la revendication 5 ou 6, **caractérisé en ce que** l'anion Y⁻ est choisi dans le groupe constitué par fluorure, hydroxyde, carboxylate, carbonate et α-hydroxycarboxylate.

8. Procédé selon la revendication 5, **caractérisé en ce que** l'anion Y⁻ est choisi dans le groupe constitué par formiate, acétate, propionate et 2-éthylhexanoate.

9. Utilisation de polyisocyanates, obtenus par un procédé selon l'une quelconque des revendications précédentes, dans des agents de revêtement, des apprêts, des charges, des vernis de surface pigmentés, des vernis de base et des vernis transparents dans le domaine du vernissage de réparation automobile ou de poids lourds, dans le vernissage de réparation automobile et de poids lourds, ainsi que pour les véhicules utilitaires dans le domaine agricole et le bâtiment.
